(19) Europäisches Patentamt
European Patent Office
Office européen des brevets

(11) Veröffentlichungsnummer : **0 471 227 B1**

# (12) EUROPÄISCHE PATENTSCHRIFT

(45) Veröffentlichungstag der Patentschrift : **02.02.94 Patentblatt 94/05**

(51) Int. Cl.$^5$ : **C07C 45/51,** C07C 49/453, B01J 23/78

(21) Anmeldenummer : **91112560.7**

(22) Anmeldetag : **26.07.91**

## (54) Verfahren zur Herstellung von Tricyclo[5.2.1.02,6]decan-8(9)-on.

(30) Priorität : **11.08.90 DE 4025526**

(43) Veröffentlichungstag der Anmeldung : **19.02.92 Patentblatt 92/08**

(45) Bekanntmachung des Hinweises auf die Patenterteilung : **02.02.94 Patentblatt 94/05**

(84) Benannte Vertragsstaaten : **AT BE CH DE DK ES FR GB IT LI LU NL SE**

(56) Entgegenhaltungen :
**DE-B- 2 047 437**
**DE-C- 953 076**

(73) Patentinhaber : **HOECHST AKTIENGESELLSCHAFT**
**D-65926 Frankfurt (DE)**

(72) Erfinder : **Bach, Hanswilhelm, Dr.**
**Alleestrasse 56**
**W-4100 Duisburg 11 (DE)**
Erfinder : **Kohl, Werner, Dr.**
**Am Walde 4**
**W-4200 Oberhausen 11 (DE)**
Erfinder : **Deckers, Gregor, Dr.**
**Reeser Strasse 23**
**W-4232 Xanten 3 (DE)**
Erfinder : **Kampmann, Detlef, Dr.**
**Friedhofstrasse 100**
**W-4630 Bochum (DE)**
Erfinder : **Kniep, Claus**
**Rosenstrasse 93**
**W-4200 Oberhausen 1 (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Tricyclo[5.2.1.$0^{2,6}$]decan-8(9)-on durch Umsetzung von 8(9)-Hydroxy-tricyclo[5.2.1.$0^{2,6}$]dec-3-en mittels eines Nickel und Magnesiumoxid enthaltenden Trägerkatalysators bei erhöhter Temperatur und gegebenenfalls erhöhtem Druck. Aller Wahrscheinlichkeit nach handelt es sich bei dieser Umsetzung um eine intramolekulare Wasserstoffumlagerung, wobei die in 8- bzw. 9-Position befindliche Hydroxylgruppe unter Wasserstoffabspaltung in die entsprechende Ketogruppe umgewandelt wird und der freigesetzte Wasserstoff an die in 3-Stellung befindliche Kohlenstoff-Kohlenstoff-Doppelbindung angelagert wird. Das nachstehende Formelschema belegt die erfindungsgemäße Umsetzung, die zur Bildung eines Ketons unter gleichzeitiger Aufhebung der im Molekül vorhandenen Kohlenstoff-Kohlenstoff-Doppelbindung führt.

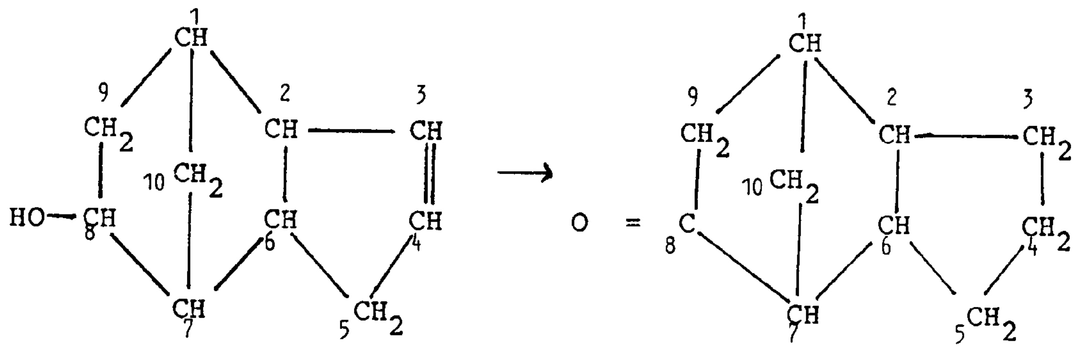

(I) 8(9)-Hydroxy-tricyclo [5.2.1.$0^{2,6}$] dec-3-en

(II) Tricyclo [5.2.1.$0^{2,6}$] decan-8(9)-on

Der für die Umsetzung als Ausgangsstoff benötigte ungesättigte Alkohol (I) kann auch in technischem Maßstab durch Wasseranlagerung an Dicyclopentadien hergestellt werden. Als Katalysatoren dienen bevorzugt $H_2SO_4$ oder Kationenaustauscher. Das gesättigte tricyclische Keton (II) kann man im weitesten Sinne zu den Terpen-Ketonen zählen. Aufgrund seiner charakteristischen Struktur und seines typischen Geruches eignet es sich als Komponente zur Herstellung von Riechstoffen. Dies trifft sowohl für das Keton selbst als auch für leicht herstellbare Derivate desselben zu. Infolge der Verwendung als Riechstoff werden an die Reinheit des gesättigten tricyclischen Ketons (II) hohe Anforderungen gestellt, wobei es sich günstig auswirkt, daß nicht umgesetzter ungesättigter Alkohol (I) durch fraktionierte Destillation abgetrennt und für eine erneute Umsetzung wiederverwendet werden kann.

Aus der DE-PS 9 53 076 ist ein Verfahren bekannt zur Umlagerung von 8-Oxytricyclodecen-(4), das dem ungesättigten tricyclischen Alkohol (I) gleichzusetzen ist, in 8-Ketotricyclodecan, das dem gesättigten tricyclischen Keton (II) entspricht, bei Temperaturen von 100 bis 250°C mittels Metallen der VIII. und bzw. oder I. Nebengruppe des periodischen Systems, die durch Metalloxide der II. Hauptgruppe des periodischen Systems aktiviert und gegebenenfalls auf inerten Trägerstoffen, vorzugsweise auf Kieselgur niedergeschlagen sind. Es hat sich jedoch herausgestellt, daß nicht jeder beliebige dieser Katalysatoren, insbesondere nicht jeder Nickel-Magnesiumoxid-Kieselgurkatalysator - wie Versuche belegen - gleichermaßen für die Durchführung dieser Reaktion geeignet ist.

Es bestand daher die Aufgabe, ein Verfahren zu entwickeln, das mittels eines technisch leicht zugänglichen Katalysators sicherstellt, daß die Umsetzung stets in reproduzierbarem Umfange und möglichst unter Vermeidung unerwünschter Nebenprodukte abläuft.

Diese Aufgabe wird gelöst durch ein Verfahren zur Herstellung von Tricyclo[5.2.1.$0^{2,6}$]decan-8(9)-on durch Umsetzung von 8(9)-Hydroxy-tricyclo[5.2.1.$0^{2,6}$]dec-3-en mittels eines Nickel und Magnesiumoxid enthaltenden Trägerkatalysators bei erhöhter Temperatur und gegebenenfalls erhöhtem Druck. Es ist dadurch gekennzeichnet, daß der Trägerkatalysator Magnesium und Nickel in einem Mol-Verhältnis von (0,0075 bis 0,075) : 1 und 17,0 bis 60,0 Gramm eines in Wasser unlöslichen Trägers je Mol Ni enthält, die aktive Nickelmetalloberfläche 110 bis 180 $m^2/g$ Nickel beträgt und 65 bis 97 % der BET-Gesamtoberfläche von Poren eines Radius $r_p \leqq 2{,}5$ nm gebildet werden.

Ein wesentliches Merkmal des verwendeten Trägerkatalysators ist die enge Verteilung der Porenradien,

ausgedrückt durch einen hohen Anteil der BET-Gesamtoberfläche, der auf Poren eines Radius $r_p \leqq 2,5$ nm zurückgeht. Unter BET-Gesamtoberfläche wird die durch Adsorption von Stickstoff nach der Brunauer, Emmett und Teller-Methode (BET) ermittelte Oberfläche verstanden.

Die BET-Gesamtoberfläche beträgt 160 bis 450, insbesondere 180 bis 380, bevorzugt 200 bis 350 $m^2/g$ Katalysator. Das Verfahren zur Bestimmung der BET-Gesamtoberfläche nach Brunauer, Emmett und Teller ist in J. Amer. Chem. Soc. 60, (1938) 309 beschrieben.

Durch die vorstehend genannte enge Verteilung der Porenradien mit Schwerpunkt auf einen Bereich $r_p \leqq$ 2,5 nm erhält der Katalysator besondere Eigenschaften, die eine Erhöhung der Aktivität und eine Verbesserung der Selektivität der dem erfindungsgemäßen Verfahren zugrundeliegenden Umsetzung zur Folge hat.

Ein weiteres Merkmal des Katalysators ist seine Zusammensetzung, ausgedrückt durch das Molverhältnis Mg zu Ni. Der Vollständigkeit halber sei auf die nachfolgende Begriffsbestimmung aufmerksam gemacht. 1 Mol Mg bzw. 1 Mol $Mg^{2+}$ entspricht 24,305 g Mg bzw. 24,305 g $Mg^{2+}$ und 1 Mol Ni bzw. 1 Mol $Ni^{2+}$ entspricht 58,71 g Ni bzw. 58,71 g $Ni^{2+}$. In reduziertem Zustand des Katalysators liegt Magnesium als $Mg^{2+}$ und Nickel überwiegend oder fast vollständig in metallischer Form vor. Unter Molverhältnis ist somit, genauer formuliert, das Verhältnis Mol $Mg^{2+}$ : (Mol Ni + Mol nichtreduziertes $Ni^{2+}$) zu verstehen.

Der Katalysator enthält in reduzierter Form, wie bereits zuvor erwähnt, Mg und Ni in einem Molverhältnis von (0,0075 bis 0,075) : 1. Besonders bewährt hat es sich, ein Molverhältnis Mg : Ni von (0,015 bis 0,060) : 1, insbesondere (0,022 bis 0,055) : 1 zu wählen.

Die Zusammensetzung des neuen Katalysators ist ferner bestimmt durch das Verhältnis der Gewichtsteile des in Wasser unlöslichen Trägers zu Ni, wobei wiederum die Summe aus Mol metallischem Nickel und Mol $Ni^{2+}$ gemeint ist.

Als Träger eignen sich verschiedene, in Wasser unlösliche Materialien. Hierunter fallen Silikate wie Ca-, Mg- und/oder Al-silikate, $Al_2O_3$, $SiO_2$ und/oder Kieselgur. Als besonders brauchbare Träger sind Mg-silikate (insbesondere in Form von Bimsstein), $Al_2O_3$, $SiO_2$ und/oder Kieselgur, insbesondere Bimsstein, $Al_2O_3$, $SiO_2$ und/oder Kieselgur, bevorzugt $SiO_2$ und/oder Kieselgur hervorzuheben. Besonders bewährt hat sich Kieselgur.

Das Trägermaterial soll üblicherweise in feinteiliger Form vorliegen. Seine Teilchen sollen eine Korngröße von 1 bis 30, insbesondere 2 bis 25, bevorzugt 3 bis 20 µm aufweisen.

Der Katalysator enthält 17,0 bis 60,0, insbesondere 25 bis 50, bevorzugt 30 bis 40 Gramm Träger je Mol Ni. Unter Mol Ni ist - wie bereits zuvor erwähnt - die Summe aus Nickel in reduzierter und nichtreduzierter Form zu verstehen.

Die aktive Nickelmetalloberfläche des Katalysators beträgt 110 bis 180, insbesondere 125 bis 160, bevorzugt 130 bis 150 $m^2/g$ Ni. Ihre Bestimmung erfolgt in Anlehnung an eine in Journal of Catalysis 81, (1983) 204 und 96, (1985) 517 näher beschriebene Methode durch Messung der durch Chemisorption bei 20°C aufgenommenen Menge Wasserstoff.

65 bis 97, insbesondere 70 bis 95, bevorzugt 75 bis 95 %, der BET-Gesamtoberfläche werden von Poren eines Radius $r_p \leqq 2,5$ nm (25 Å) gebildet. Die Bestimmung der Porenradien erfolgt nach einer in S.J. Gregg and K.S.W. Sing, Adsorption Surface Area and Porosity (Academic Press New York-London 1967), Seiten 160 bis 182, näher beschriebenen Methode.

Der Katalysator zeichnet sich ferner dadurch aus, daß 60 bis 95, insbesondere 70 bis 95, bevorzugt 73 bis 90 % der BET-Gesamtoberfläche von Poren mit einem Radius $r_p$ von 1,5 bis 2,5 nm (15 bis 25 Å) gebildet werden.

Poren mit einem Radius $r_p$ von 1,8 bis 2,5 nm (18 bis 25 Å) bilden 35 bis 85, insbesondere 45 bis 76, bevorzugt 50 bis 70 % der BET-Gesamtoberfläche.

Das Verfahren zur Herstellung der verwendeten Katalysatoren geht von einer Nickel- und Magnesiumsalze enthaltenden, wäßrigen Lösung aus. Diese Mischsalzlösung enthält 10 bis 100, insbesondere 20 bis 80, bevorzugt 30 bis 50 g Ni/l. Sie weist Magnesium entsprechend 0,2 bis 15, insbesondere 0,5 bis 12, bevorzugt 1 bis 10 g MgO/l auf.

Die Mischsalzlösung stellt man her, indem man wasserlösliche, anorganische, organische oder komplexe Salze von Nickel und Magnesium in Wasser löst. Gut geeignete Salze sind die Sulfate, Chloride, Acetate, Propionate, Butyrate und Nitrate. Besonders bewährt hat es sich, Nickel und Magnesium in Form ihrer Sulfate, Chloride, Acetate und Nitrate, bevorzugt in Form ihrer Nitrate einzusetzen.

Um einer unerwünschten Hydrolyse vorzubeugen und die Fällung günstig zu beeinflussen, empfiehlt es sich, in der Mischsalzlösung einen Überschuß freier Säure zuzulassen.

Die Mischsalzlösung wird getrennt, aber gleichzeitig mit einer wäßrigen Lösung eines basischen Fällungsmittels einem in Wasser suspendierten Träger zugeführt.

Als Fällungsmittel dient eine wäßrige Lösung einer basischen Verbindung. Besonders geeignet ist eine $Na_2CO_3$ und/oder $NaHCO_3$ enthaltende wäßrige Lösung. Das Fällungsmittel soll einen pH-Wert von 7,5 bis 13, insbesondere 8 bis 12, bevorzugt 9 bis 11 aufweisen.

Die wäßrige Lösung enthält 0,1 bis 4,0, insbesondere 0,6 bis 3,0, bevorzugt 1,6 bis 2,4 Äquivalente basische Verbindung/l Lösung. Recht gute Ergebnisse werden mit wäßrigen Lösungen, die 0,3 bis 1,5, insbesondere 0,8 bis 1,2 Mol Alkalicarbonat/l Lösung enthalten, erzielt.

Um eine möglichst vollständige Fällung sicherzustellen und zugleich ein besonders homogenes, aus basischen Nickel- und Magnesiumverbindungen bestehendes Copräzipitat zu erhalten, setzt man die basische Verbindung in geringem Überschuß ein, bezogen auf die zur vollständigen Fällung von Ni und Mg erforderliche Menge basische Verbindung.

Die Fällung wird herbeigeführt, indem man die Mischsalzlösung und das Fällungsmittel getrennt, aber gleichzeitig, kontinuierlich oder diskontinuierlich einem in Wasser suspendierten, für die Herstellung des Katalysators geeigneten Trägermaterial unter Vermischung zuführt.

Als Trägermaterial lassen sich die bereits erwähnten Stoffe, nämlich Silikate des Calciums, des Magnesiums und/oder Aluminiums, $Al_2O_3$, $SiO_2$ und/oder Kieselgur, insbesondere Kieselgur verwenden.

Die Fällung des aus basischen Nickel- und Magnesiumverbindungen bestehenden Copräzipitats wird durch vergleichsweise langsame Zugabe der Mischsalzlösung und des Fällungsmittels herbeigeführt. Die Fällungszeit sollte wenigstens 10, insbesondere wenigstens 15, bevorzugt wenigstens 20 Minuten betragen.

Man arbeitet während der Fällung bei einem konstanten pH-Wert innerhalb eines pH-Bereiches von 6 bis 8,5, insbesondere 6,5 bis 7,8. Schwankungen des pH-Wertes sollten möglichst gering gehalten werden.

Die Fällung wird bei Temperaturen oberhalb 80°C, insbesondere in einem Bereich von 90 bis 110, bevorzugt 95 bis 105°C durchgeführt.

Das Copräzipitat enthält Magnesium und Nickel entsprechend einem Molverhältnis von (0,02 bis 0,25) : 1, insbesondere (0,03 bis 0,2) : 1, bevorzugt (0,035 bis 0,1) : 1. Es wird nach Beendigung der Fällung von der Mutterlauge abgetrennt. Dies kann durch Dekantieren und/oder Filtrieren erfolgen.

Anschließend wird das Copräzipitat mit Wasser gewaschen, wobei außer den in Lösung befindlichen Bestandteilen, z.B. Na+ und $NO_3^-$, die im Copräzipitat enthaltenen, basischen Magnesiumverbindungen aus dem Copräzipitat herausgelöst werden. Dies hat zur Folge, daß sich das Molverhältnis Magnesium zu Nickel verändert und infolge der Abnahme von Magnesium zu niedrigeren Werten verschiebt. Man wäscht bei relativ hohen Temperaturen von 60 bis 90, insbesondere 65 bis 85, bevorzugt 70 bis 80°C.

Die Dauer des Waschvorganges muß ausreichend bemessen werden. Sie sollte mindestens 60, insbesondere mindestens 80, bevorzugt mindestens 90 Minuten betragen.

Falls gewünscht, kann man die gewaschene Katalysatorzusammensetzung in stückige Form bringen. Zur Formgebung kann man sich bewährter Verfahren, beispielsweise der Strangextrusion bedienen.

Die Trocknung wird bei erhöhten Temperaturen, vorzugsweise bei steigenden Temperaturen stufenweise durchgeführt. Es erweist sich als ausreichend, die Trocknung bei Temperaturen von 50 bis 120, insbesondere 55 bis 100, bevorzugt 60 bis 90°C unter Anwendung üblicher Verfahren, wie Anordnung des Trockengutes im Festbett oder im bewegten Bett, beispielsweise in Wirbelschicht, durchzuführen.

Die Reduktion der Katalysatorzusammensetzung erfolgt mittels Wasserstoff oder Wasserstoff enthaltenden Gasgemischen bei Temperaturen von 260 bis 400, insbesondere 280 bis 360°C.

Man führt das erfindungsgemäße Verfahren bei 150 bis 300, insbesondere 180 bis 280, bevorzugt 200 bis 260°C und bei 0,1 bis 1,0, insbesondere 0,15 bis 0,8, bevorzugt 0,15 bis 0,6 MPa durch.

Das erfindungsgemäße Verfahren läßt sich sowohl kontinuierlich als auch diskontinuierlich durchführen. Bei kontinuierlicher Arbeitsweise verwendet man üblicherweise einen druckfesten Rohrreaktor, der den Katalysator in stückiger Form, beispielsweise als Festbett angeordnet, enthält. Der ungesättigte tricyclische Alkohol (I) wird dem Reaktor entweder am Kopf oder am Boden zugeführt. Je nach Art der Zugabe spricht man von Riesel- oder Sumpffahrweise.

Es ist jedoch auch möglich auf den Gebrauch aufwendig konstruierter Rohrreaktoren zu verzichten und die Umsetzung beispielsweise in Rührbehältern oder Schlaufenreaktoren durchzuführen. In diesem Falle läuft die Reaktion in Gegenwart eines aufgeschlämmten Katalysators ab. Dieses Verfahren nennt man auch Suspensionsfahrweise. Hierbei wird der Katalysator in stückiger Form, beispielsweise als Fadenkorn, Tabletten, Pellets oder Granulat, aber auch in zerkleinertem Zustand oder als Pulver eingesetzt. Der Körnungsgrad sollte nicht zu grob, aber auch nicht zu fein sein. Zu große Katalysatorpartikel wirken sich wegen einer relativ geringen Oberfläche, die der Reaktion zu Verfügung steht, ungünstig aus. Hingegen erweisen sich feinteilige Katalysatoren aufgrund einer relativ großen Oberfläche als reaktiv, behindern aber wegen geringer Teilchengröße ihre durch Sedimentation, Zentrifugieren oder Filtration herbeizuführende Abtrennung. Die Entfernung des Nickel enthaltenden Katalysators nach Abschluß der Umsetzung ist notwendig, um unerwünschte Nebenreaktionen bei der Reindarstellung zu vermeiden. Während der Destillation können bereits geringe Nickelanteile beispielsweise zu Umlagerungen und Spaltungen führen. Die dabei gebildeten Nebenprodukte verunreinigen das gewünschte gesättigte, tricyclische Keton (II).

Die vorstehend beschriebene Suspensionsfahrweise eignet sich insbesondere für eine diskontinuierliche Durchführung des erfindungsgemäßen Verfahrens. Hierbei wird der Katalysator vor Beginn der Reaktion in einem Lösungmittel suspendiert. Dieses Solvens soll gegenüber der ablaufenden Umsetzung inert sein, das heißt es darf nicht in die intramolekulare Wasserstoffumlagerung eingreifen und auch nicht mit dem umzusetzenden Alkohol und dessen Reaktionsprodukt, nämlich dem Keton, reagieren.

Als Lösungsmittel sind aliphatische, cycloaliphatische, aromatische Kohlenwasserstoffe, Ether und/oder Alkohole geeignet. Der Siedepunkt dieser Lösungsmittel sollte allerdings nicht zu niedrig gewählt werden, um einen Druckaufbau während der bei vergleichsweise hohen Temperaturen ablaufenden Reaktion zu vermeiden.

Nach einer bevorzugten Variante wird auf ein Lösungsmittel der vorstehend genannten Art verzichtet und statt dessen der Katalysator in dem Ausgangsstoff (I), in dem Reaktionsgemisch und/oder dem Reaktionsprodukt (II) aufgeschlämmt.

Bei diskontinuierlicher Arbeitsweise versetzt man den aufgeschlämmten Katalysator mit der gesamt umzusetzenden Menge des ungesättigten tricyclischen Alkohols (I), erhitzt unter Rühren bis zu der gewünschten Temperatur und läßt reagieren. Nach Beendigung der Reaktion läßt man abkühlen und trennt das Reaktionsprodukt vom Katalysator ab. Der abgetrennte Katalysator kann in die Reaktion erneut eingesetzt werden.

Üblicherweise zerfallen suspendierte Hydrierkatalysatoren bei längerem Gebrauch in zunehmendem Maße. Die daraus resultierenden, feinteiligen Partikel sind nicht erwünscht, da sie - wie zuvor erwähnt - die Abtrennung des suspendierten Katalysators nach der Umsetzung erschweren. Üblicherweise entfernt man den aufgeschlämmten Katalysator durch Sedimentation und/oder Zentrifugieren und/oder Filtration. Je feiner jedoch die Katalysatorpartikel sind, desto schwerer lassen sie sich aus dem Reaktionsgemisch entfernen. Eine geringere Teilchengröße bedeutet eine verminderte Sedimentationsgeschwindigkeit, was sich auch auf das Zentrifugieren nachteilig auswirkt. Verwendet man die Filtration zur Katalysatorabtrennung, so führen besonders kleine Teilchen infolge Verlegung der Filterporen rasch zu einer Blockierung der Filtrationseinheit. Dadurch kommt es zu einem Druckanstieg in der Apparatur, mit der Folge, daß die Filtration unterbrochen werden muß, um Verstopfungen zu beseitigen.

Der verwendete Hydrierkatalysator weist eine vergleichsweise geringe Neigung zum Zerfallen auf und läßt sich daher selbst bei wiederholtem Einsatz ohne Schwierigkeiten aus dem Reaktionsgemisch abtrennen.

Aus diesem Grund hat es sich bewährt, den Katalysator in vorzerkleinertem Zustand einzusetzen.

Die nachfolgenden Beispiele belegen die Erfindung, ohne sie zu beschränken.

Beispiel

400 g 8(9)-Hydroxy-tricyclo[5.2.1.$0^{2,6}$]-dec-3-en werden in einem mit $N_2$ gespülten Rührautoklav (Volumen 1 Liter) vorgelegt. 5 Gew.-% eines Katalysators, der je Mol Nickel 38 g Träger (Kieselgur), ein Molverhältnis Mg : Ni = 0,04 : 1, eine aktive Nickelmetalloberfläche von 130 $m^2$/g Nickel und eine BET-Gesamtoberfläche von 200 $m^2$/g Katalysator aufweist, wobei 90 % der BET-Gesamtoberfläche von Poren eines Radius $r_p \leqq 2,5$ nm (25 Å) gebildet werden, werden eingesetzt (bezogen auf 8(9)-Hydroxy-tricyclo[5.2.1.$0^{2,6}$]dec-3-en). Anschließend wird auf 235°C erhitzt. Während der Reaktionszeit von 10 Stunden stellt sich ein Druck von 0,25 bis 0,5 MPa ein.

Der Umsatz bezogen auf eingesetzten Alkohol ist >99,9 %, die Selektivität hinsichtlich der Bildung von Tricyclo[5.2.1.$0^{2,6}$]decan-8(9)-on beträgt 92,5 %. Da das ungesättigte Keton (Tricyclo[5.2.1.$0^{2,6}$]dec-3-en-8(9)-on nur in Spuren auftritt, ist eine Nachbehandlung des Reaktionsproduktes - wie in Vergleichsversuch 3 angegeben - nicht erforderlich.

Vergleichsversuch 1

Es wird wie im Beispiel angegeben gearbeitet. Bezogen auf Alkohol werden 5 Gew.-% Katalysator eingesetzt. Der Katalysator weist je Mol Nickel 26 g Träger (Kieselgur), ein Molverhältnis Mg : Ni = 0,12 : 1 und eine aktive Nickelmetalloberfläche von 65 $m^2$/g Nickel auf. Die Reaktionstemperatur beträgt 235°C. Während der 10stündigen Reaktionszeit stellt sich ein Druck von 0,16 bis 0,44 MPa ein. Der Umsatz, bezogen auf eingesetzten Alkohol, liegt bei 38,5 %, die Selektivität hinsichtlich der Bildung von Tricyclo[5.2.1.$0^{2,6}$]decan-8(9)-on beträgt lediglich 7 %. Das restliche Reaktionsgemisch weist neben nichtumgesetztem Einsatzstoff hauptsächlich 8(9)-Hydroxy-tricyclo[5.2.1.$0^{2,6}$]decan und Tricyclo[5.2.1.$0^{2,6}$]dec-3-en-8(9)-on, also einen gesättigten Alkohol und ein ungesättigtes Keton, auf.

Vergleichsversuch 2

Es wird wie im Beispiel angegeben gearbeitet. Bezogen auf Alkohol werden 5 Gew.-% Katalysator eingesetzt. Der Katalysator weist je Mol Nickel 33 g Träger (Kieselgur), ein Molverhältnis Mg : Ni = 0,12 : 1 und eine aktive Nickelmetalloberfläche von 86 $m^2/g$ Nickel auf. Die Reaktionstemperatur beträgt 235°C. Während der 10stündigen Reaktionszeit stellt sich ein Druck von 0,2 bis 0,4 MPa ein.

Der Umsatz bezogen auf eingesetzten Alkohol liegt bei 67,1 %, die Selektivität hinsichtlich der Bildung von Tricyclo[5.2.1.$0^{2,6}$]decan-8(9)-on beträgt lediglich 21 %. Das restliche Reaktionsgemisch weist dieselben Bestandteile wie im Vergleichsversuch 1 angegeben auf.

Vergleichsversuch 3

Es wird wie im Beispiel angegeben gearbeitet. Bezogen auf Alkohol werden 5 Gew.-% Katalysator eingesetzt. Der Katalysator weist je Mol Nickel 33 g Träger (Kieselgur), ein Molverhältnis Mg : Ni = 0,08 : 1 und eine aktive Nickelmetalloberfläche von 90 $m^2/g$ Nickel auf. Die Reaktionstemperatur beträgt 250°C. Während der 10stündigen Reaktionszeit stellt sich ein Druck von 0,25 bis 0,4 MPa ein.

Der Umsatz bezogen auf eingesetzten Alkohol ist >99,9 %, die Selektivität hinsichtlich der Bildung von Tricyclo[5.2.1.$0^{2,6}$]decan-8(9)-on beträgt lediglich 81,5 %.

Der Versuch, aus dem Reaktionsprodukt das gewünschte Keton destillativ abzutrennen, führt wegen der Anwesenheit des ungesättigten Ketons (Tricyclo[5.2.1.$0^{2,6}$]dec-3-en-8(9)-on), dessen Siedepunkt sehr nahe bei dem des Wertproduktes liegt, nicht zum Erfolg. Um das Wertprodukt (Tricyclo[5.2.1.$0^{2,6}$]decan-8(9)-on) in der erforderlichen Reinheit zu gewinnen, muß das Reaktionsgemisch einer separaten Nachhydrierung in Gegenwart eines Edelmetallkatalysators unterworfen werden.

**Patentansprüche**

1. Verfahren zur Herstellung von Tricyclo[5.2.1.$0^{2,6}$]decan-8(9)-on durch Umsetzung von 8(9)-Hydroxytricyclo[5.2.1.$0^{2,6}$]dec-3-en mittels eines Nickel und Magnesiumoxid enthaltenden Trägerkatalysators bei erhöhter Temperatur und gegebenenfalls erhöhtem Druck, dadurch gekennzeichnet, daß der Trägerkatalysator Mg und Ni in einem Molverhältnis von (0,0075 bis 0,075) : 1 und 17,0 bis 60,0 Gramm eines in Wasser unlöslichen Trägers je Mol Ni enthält, die aktive Nickelmetalloberfläche 110 bis 180 $m^2/g$ Ni beträgt und 65 bis 97 % der BET-Gesamtoberfläche von Poren eines Radius $r_p \leqq 2,5$ nm gebildet werden.

2. Verfahren nach Anspruch 1, dadurch gekennzeichnet, daß die BET-Gesamtoberfläche des Trägerkatalysators 160 bis 450, insbesondere 180 bis 380, bevorzugt 200 bis 350 $m^2/g$ Katalysator beträgt.

3. Verfahren nach Anspruch 1 oder 2, dadurch gekennzeichnet, daß der Trägerkatalysator Mg und Ni in einem Molverhältnis von (0,015 bis 0,06) : 1, insbesondere (0,022 bis 0,055) : 1 enthält.

4. Verfahren nach einem oder mehreren der Ansprüche 1 bis 3, dadurch gekennzeichnet, daß der Trägerkatalysator Ca-, Mg-, Al-silikat, $Al_2O_3$, $SiO_2$ und/oder Kieselgur, insbesondere $Al_2O_3$, $SiO_2$ und/oder Kieselgur, bevorzugt $SiO_2$ und/oder Kieselgur, besonders bevorzugt Kieselgur als Träger enthält.

5. Verfahren nach einem oder mehreren der Ansprüche 1 bis 4, dadurch gekennzeichnet, daß die Umsetzung bei 150 bis 300, insbesondere 180 bis 280, bevorzugt 200 bis 260°C durchgeführt wird.

6. Verfahren nach einem oder mehreren der Ansprüche 1 bis 5, dadurch gekennzeichnet, daß die Umsetzung unter einem Druck von 0,1 bis 1, insbesondere 0,15 bis 0,8, bevorzugt 0,15 bis 0,6 MPa durchgeführt wird.

**Claims**

1. A process for the preparation of tricyclo-[5.2.1.$0^{2,6}$]decan-8(9)-one by the conversion of 8(9)-hydroxytricyclo[5.2.1.$0^{2,6}$]dec-3-ene by means of a supported catalyst containing nickel and magnesium oxide at an elevated temperature and, if necessary, an elevated pressure, wherein the supported catalyst contains Mg and Ni in a molar ratio of (0.0075 to 0.075) : 1 and 17.0 to 60.0 grams of a water-insoluble support

per mole of Ni, the active nickel metal surface area being 110 to 180 $m^2/g$ of Ni, and 65 to 97% of the BET total surface area is composed of pores having a radius $r_p \leqq 2.5$ nm.

2. The process as claimed in claim 1, wherein the BET total surface area of the supported catalyst is 160 to 450, in particular 180 to 380 and preferably 200 to 350, $m^2/g$ of catalyst.

3. The process as claimed in claim 1 or 2, wherein the supported catalyst contains Mg and Ni in a molar ratio of (0.015 to 0.06) : 1, in particular (0.022 to 0.055) : 1.

4. The process as claimed in one or more of claims 1 to 3, wherein the supported catalyst contains, as the support, Ca silicate, Mg silicate, Al silicate, $Al_2O_3$, $SiO_2$ and/or kieselguhr, in particular Al2O3, $SiO_2$ and/or kieselguhr, preferably $SiO_2$ and/or kieselguhr and particularly preferably kieselguhr.

5. The process as claimed in one or more of claims 1 to 4, wherein the conversion is carried out at 150 to 300°C, in particular 180 to 280°C and preferably 200 to 260°C.

6. The process as claimed in one or more of claims 1 to 5, wherein the conversion is carried out under a pressure of 0.1 to 1, in particular 0.15 to 0.8 and preferably 0.15 to 0.6, MPa.

## Revendications

1. Procédé de préparation de la tricyclo[5.2.1.$0^{2,6}$]décan-8(9)-one par conversion du 8(9)-hydroxy-tricyclo[5.2.1.$0^{2,6}$]déc-3-ène au moyen d'un catalyseur sur support contenant du nickel et de l'oxyde de magnésium, à haute température et éventuellement sous pression, caractérisé en ce que le catalyseur sur support contient Mg et Ni dans un rapport molaire de (0,0075 à 0,075): 1 et 17,0 à 60,0 g d'un support insoluble dans l'eau par mole de Ni, la surface active de nickel métallique est de 110 à 180 $m^2/g$ de Ni et 65 à 97 % de la surface BET totale sont formés par des pores d'un rayon $r_p \leqq 2,5$ nm.

2. Procédé selon la revendication 1, caractérisé en ce que la surface BET totale du catalyseur sur support est de 160 à 450, en particulier de 180 à 380, de préférence de 200 à 350 $m^2/g$ de catalyseur.

3. Procédé selon la revendication 1 ou 2, caractérisé en ce que le catalyseur sur support contient Mg et Ni dans un rapport molaire de (0,015 à 0,06): 1, en particulier de (0,022 à 0,055): 1.

4. Procédé selon une ou plusieurs des revendications 1 à 3, caractérisé en ce que le catalyseur sur support contient comme support un silicate de Ca, de Mg, de Al, $Al_2O_3$, $SiO_2$ et/ou du kieselguhr, en particulier $Al_2O_3$, $SiO_2$ et/ou du kieselguhr, de préférence $SiO_2$ et/ou du kieselguhr, de préférence encore du kieselguhr.

5. Procédé selon une ou plusieurs des revendications 1 à 4, caractérisé en ce que la réaction est réalisée à une température de 150 à 300, en particulier de 180 à 280, de préférence de 200 à 260°C.

6. Procédé selon une ou plusieurs des revendications 1 à 5, caractérisé en ce que la réaction est réalisée sous une pression de 0,1 à 1, en particulier de 0,15 à 0,8, de préférence de 0,15 à 0,6 MPa.